# EUROPEAN PATENT APPLICATION

(11) **EP 4 506 955 A1**
(43) Date of publication of application: **12.02.2025**
(21) Application number: 23190715.5
(22) Date of filing: 10.08.2023
(51) Int. Cl.: G16H 40/63, G16H 40/67, G16H 80/00, A61B 5/00

(54) **METHOD AND SYSTEM FOR PERFORMING A DATA EXCHANGE**

(71) Applicant: BIOTRONIK SE & Co. KG, 12359 Berlin (DE)
(72) Inventor: Krüger, Daniel, 15741 Bestensee (DE); Müller, Jens, 14195 Berlin (DE); Deutschmann, Hendrik, 10247 Berlin (DE)
(74) Representative: Biotronik Corporate Services SE

(57) **Abstract**

The invention relates to a computer-implemented method and system for performing a data exchange between a programming instance (10) for an IMD (12) and a HIS (14), comprising the steps of providing (S1) configuration information (C) for the data exchange between the programming instance for the IMD and the HIS, said configuration information comprising a second data format and/or protocol (18a, 18b) supported by the HIS; providing (S2) first medical data (D1) acquired via the IMD by the programming instance, said first medical data having a first data format and/or protocol (16a, 16b) supported by the programming instance; converting (S3) the first medical data comprising the first data format and/or protocol supported by the programming instance to first medical data comprising the second data format and/or protocol supported by the HIS; and transmitting (S4) the converted first medical data to the HIS.

## Description

The invention relates to a computer-implemented method for performing a data exchange between a programming instance for an implantable medical device (IMD) and a hospital information system (HIS).

Furthermore, the invention relates to a system for performing a data exchange between a programming instance for an implantable medical device and a hospital information system.

Currently, when importing medical data from a programming instance for an implantable medical device such as a programmer or an app on a patient communication device said programming instance provides the data using certain configuration information supported by the specific programming instance.

Said configuration information may comprise a data format and/or a protocol for transmitting the medical data from the programming instance for the implantable medical device to the hospital information system and vice versa.

The hospital information system often times does not support the configuration information used by the programming instance but instead supports a different data format and/or a different protocol for receiving the medical data from the programming instance for the implantable medical device and vice versa, i.e. transmitting data to the programming instance for the implantable medical device. Known solutions hence require manual data import steps or complicated hardware installations in clinic IT infrastructures.

It is therefore an object of the present invention to provide an improved method for performing a data exchange between a programming instance for an implantable medical device and a hospital information system such that a more user-friendly configuration of the data transfer between the programming instance for the implantable medical device and the hospital information system can be achieved.

The object is solved by a computer-implemented method for performing a data exchange between a programming instance for an implantable medical device and a hospital information system having the features of claim 1.

Furthermore, the object is solved by a system for performing a data exchange between a programming instance for an implantable medical device and a hospital information system having the features of claim 10.

Moreover, the object is solved by computer program having the features of claim 14 and by a computer-readable data carrier having the features of claim 15.

Further developments and advantageous embodiments are defined in the dependent claims.

The present invention provides a computer-implemented method for performing a data exchange between a programming instance for an implantable medical device and a hospital information system.

The method comprises providing configuration information for the data exchange between the programming instance for the implantable medical device and the hospital information system, said configuration information comprising a second data format and/or a second protocol supported by the hospital information system.

Furthermore, the method comprises providing first medical data acquired via the implantable medical device by the programming instance for the implantable medical device, said first medical data having a first data format and/or a first protocol supported by the programming instance.

The method moreover comprises converting the first medical data comprising the first data format and/or the first protocol supported by the programming instance to first medical data comprising the second data format and/or the second protocol supported by the hospital information system, and transmitting the converted first medical data to the hospital information system.

In addition, the present invention provides a system for performing a data exchange between a programming instance for an implantable medical device and a hospital information system.

The system comprises means for providing configuration information for the data exchange between the programming instance for the implantable medical device and the hospital information system, said configuration information comprising a second data format and/or a second protocol supported by the hospital information system.

In addition, the system comprises the programming instance for the implantable medical device configured to provide first medical data acquired via the implantable medical device, said first medical data having a first data format and/or a first protocol supported by the programming instance.

Moreover, the system comprises means for converting the first medical data comprising the first data format and/or the first protocol supported by the programming instance to first medical data comprising the second data format and/or the second protocol supported by the hospital information system, and means for transmitting the converted first medical data to the hospital information system.

Moreover, the present invention provides a computer program with program code to perform the methods of the present invention when the computer program is executed on a computer. In addition, the present invention provides a computer-readable data carrier containing program code of a computer program for performing the methods of the present invention when the computer program is executed on a computer.

An idea of the present invention is to provide an enhanced method and system for configuring a data exchange between the programming instance for an implantable medical device and the hospital information system and subsequently performing said data exchange.

By performing a pre-configuration of the programming instance and/or a conversion means of an IT service provider and providing conversion of the first medical data comprising the first data format and/or the first protocol supported by the programming instance to first medical data comprising the second data format and/or the second protocol supported by the hospital information system a data transfer from the programming instance of the implantable medical device to the hospital information system and vice versa can be performed efficiently and securely without the need for any manual configuration steps.

This invention thus eases interoperability of programming instances and hospital information systems. A multitude of programming instances of different device manufacturers can thus be made compatible with a multitude of hospital information systems and vice versa hence providing an improved integration and interoperability of the overall system comprising the programming instances and hospital information system.

According to an aspect of the invention, the first medical data comprising the first data format and/or the first protocol supported by the programming instance is converted to first medical data comprising the second data format and/or the second protocol supported by the hospital information system by a first conversion means of the programming instance for the implantable medical device.

The programming instance for the implantable medical device thus advantageously is capable of converting itself the data from the first data format and/or the first protocol to the second data format and/or the second protocol.

According to a further aspect of the invention, the first medical data comprising the first data format and/or the first protocol supported by the programming instance is converted to first medical data comprising the second data format and/or the second protocol supported by the hospital information system by a second conversion means, in particular a conversion service, of an IT service provider.

By using said second conversion means, in particular a conversion service, of the IT service provider placing a computational load on the programming instance for the implantable medical device can advantageously be avoided. In doing so, a faster conversion of the data can be achieved since the second conversion means comprises more computational power than the programming instance for the implantable medical device.

According to a further aspect of the invention, the first medical data comprising the first data format and/or the first protocol supported by the programming instance is transmitted from the programming instance for the implantable medical device to the second conversion means of the IT service provider, wherein after conversion of the first medical data to the second data format and/or the second protocol supported by the hospital information system the first medical data is sent back to the programming instance for the implantable medical device.

Thus, the conversion of the data format and the protocol is advantageously outsourced to the IT service provider, wherein conversion takes place in an efficient, seamless manner.

According to a further aspect of the invention, the configuration information is provided via a web interface of the hospital information system to a webserver of the IT service provider, wherein the configuration information is transmitted by the webserver of the IT service provider to the programming instance for the implantable medical device or to the second conversion means of the IT service provider.

A user is thus enabled to initially specify a desired second data format and/or second protocol via the web interface of the hospital information system.

A web service (e.g. Web View) may be used with a browser by the clinic staff and/or clinic IT staff to do the configuration. Thus, a web interface is covered that may be operated directly from the KIS and a web service that may be operated by the staff.

According to a further aspect of the invention, the converted first medical data is transmitted from the programming instance for the implantable medical device to the hospital information system. The pre-configured programming instance for the implantable medical device is thus able to provide the first medical data to the hospital information system in the correct data format and/or protocol no matter where the data conversion takes place.

According to a further aspect of the invention, the method further comprises providing second medical data stored in a data storage of the hospital information system to the programming instance for the implantable medical device, said second medical data having a second data format and/or a second protocol supported by the hospital information system.

The hospital information system can thus advantageously transfer data format supported by the hospital information system without the requirement of any data conversion prior to sending the data to the programming instance for the implantable medical device.

According to a further aspect of the invention, the method further comprises converting the second medical data from the second data format and/or the second protocol supported by the hospital information system to the first data format and/or the first protocol supported by the programming instance by the first conversion means of the programming instance for the implantable medical device. The first conversion means of the programming instance for the implantable medical device thus advantageously provides bidirectional conversion capability, no matter if medical data is sent from the programming instance to the hospital information system or vice versa.

According to a further aspect of the invention, the method further comprises converting the second medical data from the second data format and/or the second protocol supported by the hospital information system to the first data format and/or the first protocol supported by the programming instance by the second conversion means of the IT service provider. The second conversion means of the IT service provider thus also advantageously provides bidirectional conversion capability, no matter if medical data is sent from the programming instance to the hospital information system or vice versa.

According to a further aspect of the invention, the programming instance for the implantable medical device is configured to convert the outputted first medical data comprising the first data format and/or the first protocol supported by the programming instance to first medical data comprising the second data format and/or the second protocol supported by the hospital information system. The programming instance for the implantable medical device thus advantageously is capable of converting itself the data from the first data format and/or the first protocol to the second data format and/or the second protocol.

According to a further aspect of the invention, a second conversion means, in particular a conversion service, of an IT service provider is configured to convert the first medical data comprising the first data format and/or the first protocol outputted be the programming instance for the implantable medical device to first medical data comprising the second data format and/or the second protocol supported by the hospital information system. By using said second conversion means, in particular a conversion service of the IT service provider, placing a computational load on the programming instance for the implantable medical device can advantageously avoided. In doing so, furthermore a faster conversion of the data can be achieved since the second conversion means comprises more computational power than the programming instance for the implantable medical device.

According to a further aspect of the invention, the second conversion means of the IT service provider is configured, after conversion of the first medical data to the second data format and/or the second protocol supported by the hospital information system, to send the first medical data back to the programming instance for the implantable medical device. Thus, the conversion of the data format and the protocol is advantageously outsourced to the IT service provider, wherein conversion takes place in an efficient, seamless manner.

The herein described features of the computer-implemented method for performing a data exchange between a programming instance for an implantable medical device and a hospital information system are also disclosed for the system for performing a data exchange between a programming instance for an implantable medical device and a hospital information system and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a flowchart of a computer-implemented method for performing a data exchange between a programming instance for an implantable medical device and a hospital information system according to the preferred embodiment of the invention; and
- Fig. 2: shows a diagram of a system for performing a data exchange between a programming instance for an implantable medical device and a hospital information system according to the preferred embodiment of the invention.

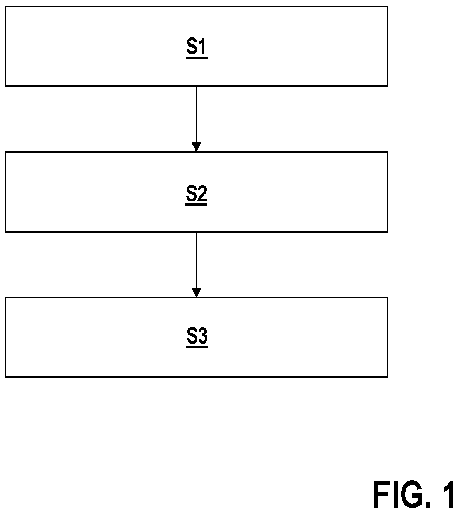

The computer-implemented method for performing a data exchange between a programming instance 10 for an implantable medical device 12 and a hospital information system 14 shown in Fig.1 comprises providing S1 configuration information C for the data exchange between the programming instance 10 for the implantable medical device 12 and the hospital information system 14, said configuration information C comprising a second data format 18a and/or a second protocol 18b supported by the hospital information system 14.

Furthermore, the method comprises providing S2 first medical data D1 acquired via the implantable medical device 12 by the programming instance 10 for the implantable medical device 12, said first medical data D1 having a first data format 16a and/or a first protocol 16b supported by the programming instance 10.

The method in addition comprises converting S3 the first medical data D1 comprising the first data format 16a and/or the first protocol 16b supported by the programming instance 10 to first medical data D1 comprising the second data format 18a and/or the second protocol 18b supported by the hospital information system 14, and transmitting S4 the converted first medical data D1 to the hospital information system 14.

The first medical data D1 comprising the first data format 16a and/or the first protocol 16b supported by the programming instance 10 is converted to first medical data D1 comprising the second data format 18a and/or the second protocol 18b supported by the hospital information system 14 by a first conversion means 19 of the programming instance 10 for the implantable medical device 12.

Alternatively, the first medical data D1 comprising the first data format 16a and/or the first protocol 16b supported by the programming instance 10 is converted to first medical data D1 comprising the second data format 18a and/or the second protocol 18b supported by the hospital information system 14 by a second conversion means 21, in particular a conversion service, of an IT service provider.

The first medical data D1 comprising the first data format 16a and/or the first protocol 16b supported by the programming instance 10 is transmitted from the programming instance 10 for the implantable medical device 12 to the second conversion means 21 of the IT service provider. After conversion of the first medical data D1 to the second data format 18a and/or the second protocol 18b supported by the hospital information system 14 the first medical data D1 is sent back to the programming instance 10 for the implantable medical device 12.

The configuration information C is provided via a web interface 20 of the hospital information system 14, in particular of a hospital infrastructure 13, via an internet connection 15 to a webserver 22 of the IT service provider. The configuration information C is transmitted by the webserver 22 of the IT service provider to the programming instance 10 for the implantable medical device 12 or to the second conversion means 21 of the IT service provider.

The webserver 22 and the second conversion means 21 are placed in an infrastructure 17 of the IT service provider.

The converted first medical data D1 is then transmitted from the programming instance 10 for the implantable medical device 12 to the hospital information system 14.

The method further comprises providing second medical data D2 stored in a data storage 14a of the hospital information system 14 to the programming instance 10 for the implantable medical device 12, said second medical data D2 having a second data format 18a and/or a second protocol 18b supported by the hospital information system 14.

In addition, the method comprises converting the second medical data D2 from the second data format 18a and/or the second protocol 18b supported by the hospital information system 14 to the first data format 16a and/or the first protocol 16b supported by the programming instance 10 by the first conversion means 19 of the programming instance 10 for the implantable medical device 12.

Alternatively, the method comprises converting the second medical data D2 from the second data format 18a and/or the second protocol 18b supported by the hospital information system 14 to the first data format 16a and/or the first protocol 16b supported by the programming instance 10 by the second conversion means 21 of the IT service provider.

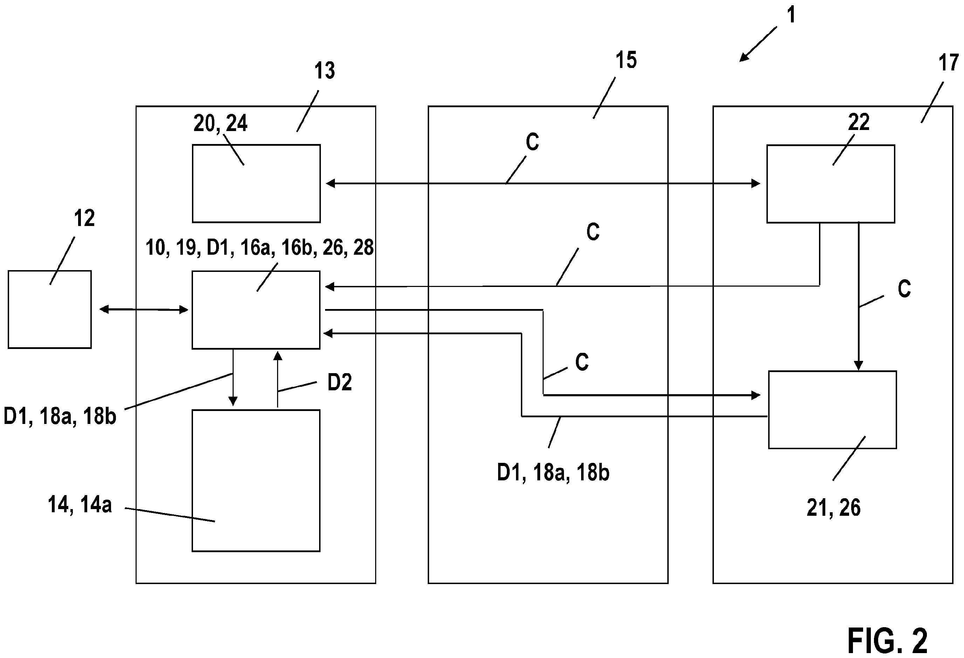

Fig. 2 shows a diagram of a system for performing a data exchange between a programming instance 10 for an implantable medical device 12 and a hospital information system according to the preferred embodiment of the invention.

The system comprises means 24 for providing S1 configuration information C for the data exchange between the programming instance 10 for the implantable medical device 12 and the hospital information system 14, said configuration information C comprising a second data format 18a and/or a second protocol 18b supported by the hospital information system 14.

Furthermore, the system comprises the programming instance 10 for the implantable medical device 12 configured to provide first medical data D1 acquired via the implantable medical device 12, said first medical data D1 having a first data format 16a and/or a first protocol 16b supported by the programming instance 10.

The system moreover comprises means 26 for converting S3 the first medical data D1 comprising the first data format 16a and/or the first protocol 16b supported by the programming instance 10 to first medical data D1 comprising the second data format 18a and/or the second protocol 18b supported by the hospital information system 14; and means 28 for transmitting S4 the converted first medical data D1 to the hospital information system 14.

The programming instance 10 for the implantable medical device 12 is configured to convert the outputted first medical data D1 comprising the first data format 16a and/or the first protocol 16b supported by the programming instance 10 to first medical data D1 comprising the second data format 18a and/or the second protocol 18b supported by the hospital information system 14.

Alternatively, a second conversion means 21, in particular a conversion service, of an IT service provider is configured to convert the first medical data D1 comprising the first data format 16a and/or the first protocol 16b outputted be the programming instance 10 for the implantable medical device 12 to first medical data D1 comprising the second data format 18a and/or the second protocol 18b supported by the hospital information system 14.

The second conversion means 21 of the IT service provider is further configured, after conversion of the first medical data D1 to the second data format 18a and/or the second protocol 18b supported by the hospital information system 14, to send the first medical data D1 back to the programming instance 10 for the implantable medical device 12.

### Reference Signs

- 1: system
- 10: programming instance
- 12: implantable medical device
- 13: hospital infrastructure
- 14: hospital information system
- 14a: data storage
- 15: internet connection
- 16a: first data format
- 16b: first protocol
- 17: infrastructure of an IT service provider
- 18a: second data format
- 18b: second protocol
- 19: first conversion means
- 20: web interface
- 21: second conversion means
- 22: webserver
- 24, 26, 28: means
- C: configuration information
- D1: first medical data
- D2: second medical data
- S1 - S3: method steps

## Claims

1. Computer-implemented method for performing a data exchange between a programming instance (10) for an implantable medical device (12) and a hospital information system (14), comprising the steps of:
providing (S1) configuration information (C) for the data exchange between the programming instance (10) for the implantable medical device (12) and the hospital information system (14), said configuration information (C) comprising a second data format (18a) and/or a second protocol (18b) supported by the hospital information system (14);
providing (S2) first medical data (D1) acquired via the implantable medical device (12) by the programming instance (10) for the implantable medical device (12), said first medical data (D1) having a first data format (16a) and/or a first protocol (16b) supported by the programming instance (10);
converting (S3) the first medical data (D1) comprising the first data format (16a) and/or the first protocol (16b) supported by the programming instance (10) to first medical data (D1) comprising the second data format (18a) and/or the second protocol (18b) supported by the hospital information system (14); and
transmitting (S4) the converted first medical data (D1) to the hospital information system (14).

2. Computer-implemented method of claim 1, wherein the first medical data (D1) comprising the first data format (16a) and/or the first protocol (16b) supported by the programming instance (10) is converted to first medical data (D1) comprising the second data format (18a) and/or the second protocol (18b) supported by the hospital information system (14) by a first conversion means (19) of the programming instance (10) for the implantable medical device (12).

3. Computer-implemented method of claim 1, wherein the first medical data (D1) comprising the first data format (16a) and/or the first protocol (16b) supported by the programming instance (10) is converted to first medical data (D1) comprising the second data format (18a) and/or the second protocol (18b) supported by the hospital information system (14) by a second conversion means (21), in particular a conversion service, of an IT service provider.

4. Computer-implemented method of claim 3, wherein the first medical data (D1) comprising the first data format (16a) and/or the first protocol (16b) supported by the programming instance (10) is transmitted from the programming instance (10) for the implantable medical device (12) to the second conversion means (21) of the IT service provider, wherein after conversion of the first medical data (D1) to the second data format (18a) and/or the second protocol (18b) supported by the hospital information system (14) the first medical data (D1) is sent back to the programming instance (10) for the implantable medical device (12).

5. Computer-implemented method of claim 3 or 4, wherein the configuration information (C) is provided via a web interface (20) of the hospital information system (14) to a webserver (22) of the IT service provider, wherein the configuration information (C) is transmitted by the webserver (22) of the IT service provider to the programming instance (10) for the implantable medical device (12) or to the second conversion means (21) of the IT service provider.

6. Computer-implemented method of any one of claims 3 to 5, wherein the converted first medical data (D1) is transmitted from the programming instance (10) for the implantable medical device (12) to the hospital information system (14).

7. Computer-implemented method of any one of claims 3 to 6, wherein the method further comprises providing second medical data (D2) stored in a data storage (14a) of the hospital information system (14) to the programming instance (10) for the implantable medical device (12), said second medical data (D2) having the second data format (18a) and/or the second protocol (18b) supported by the hospital information system (14).

8. Computer-implemented method of claim 7, wherein the method further comprises converting the second medical data (D2) from the second data format (18a) and/or the second protocol (18b) supported by the hospital information system (14) to the first data format (16a) and/or the first protocol (16b) supported by the programming instance (10) by the first conversion means (19) of the programming instance (10) for the implantable medical device (12).

9. Computer-implemented method of claim 7 or 8, wherein the method further comprises converting the second medical data (D2) from the second data format (18a) and/or the second protocol (18b) supported by the hospital information system (14) to the first data format (16a) and/or the first protocol (16b) supported by the programming instance (10) by the second conversion means (21) of the IT service provider.

10. System (1) for performing a data exchange between a programming instance (10) for an implantable medical device (12) and a hospital information system (14), comprising:
means (24) for providing (S1) configuration information (C) for the data exchange between the programming instance (10) for the implantable medical device (12) and
the hospital information system (14), said configuration information (C) comprising a second data format (18a) and/or a second protocol (18b) supported by the hospital information system (14);
the programming instance (10) for the implantable medical device (12) configured to provide first medical data (D1) acquired via the implantable medical device (12), said first medical data (D1) having a first data format (16a) and/or a first protocol (16b) supported by the programming instance (10);
means (26) for converting (S3) the first medical data (D1) comprising the first data format (16a) and/or the first protocol (16b) supported by the programming instance (10) to first medical data (D1) comprising the second data format (18a) and/or the second protocol (18b) supported by the hospital information system (14); and
means (28) for transmitting (S4) the converted first medical data (D1) to the hospital information system (14).

11. System of claim 10, wherein the programming instance (10) for the implantable medical device (12) is configured to convert the outputted first medical data (D1) comprising the first data format (16a) and/or the first protocol (16b) supported by the programming instance (10) to first medical data (D1) comprising the second data format (18a) and/or the second protocol (18b) supported by the hospital information system (14).

12. System of claim 10 or 11, wherein a second conversion means (21), in particular a conversion service, of an IT service provider is configured to convert the first medical data (D1) comprising the first data format (16a) and/or the first protocol (16b) outputted be the programming instance (10) for the implantable medical device (12) to first medical data (D1) comprising the second data format (18a) and/or the second protocol (18b) supported by the hospital information system (14).

13. System of claim 12, wherein the second conversion means (21) of the IT service provider is configured, after conversion of the first medical data (D1) to the second data format (18a) and/or the second protocol (18b) supported by the hospital information system (14), to send the first medical data (D1) back to the programming instance (10) for the implantable medical device (12).

14. Computer program with program code to perform the method of any one of claims 1 to 9 when the computer program is executed on a computer.

15. Computer-readable data carrier containing program code of a computer program for performing the method of any one of claims 1 to 9 when the computer program is executed on a computer.
